(19)

Europäisches
Patentamt
European
Patent Office
Office européen
des brevets

(11) **EP 3 235 242 B1**

(12) **EUROPEAN PATENT SPECIFICATION**

(45) Date of publication and mention
of the grant of the patent:
**29.10.2025   Bulletin 2025/44**

(21) Application number: **15870266.2**

(22) Date of filing: **14.12.2015**

(51) International Patent Classification (IPC):
*A61B 5/00* (2006.01)      *A61B 5/103* (2006.01)
*A61B 5/1455* (2006.01)

(52) Cooperative Patent Classification (CPC):
**A61B 5/1032; A61B 5/0077; A61B 5/1455;**
**A61B 5/6898; H04N 23/55; H04N 23/56;**
A61B 5/441; A61B 2562/0233; A61B 2562/185

(86) International application number:
**PCT/KR2015/013678**

(87) International publication number:
**WO 2016/099099 (23.06.2016 Gazette 2016/25)**

(54) **IMAGE CAPTURING DEVICE AND SENSING PROTECTION DEVICE**

BILDERFASSUNGSVORRICHTUNG UND ABTASTENDE SCHUTZVORRICHTUNG

DISPOSITIF DE CAPTURE D'IMAGES ET DISPOSITIF DE PROTECTION DE DÉTECTION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **15.12.2014   RU 2014150762**
**08.12.2015   KR 20150173720**

(43) Date of publication of application:
**25.10.2017   Bulletin 2017/43**

(73) Proprietor: **Samsung Electronics Co., Ltd.**
**Gyeonggi-do 16677 (KR)**

(72) Inventor: **VILENSKII, Maxim Alexeevich**
**Moscow 142770 (RU)**

(74) Representative: **HGF**
**HGF Limited**
**1 City Walk**
**Leeds LS11 9DX (GB)**

(56) References cited:
WO-A1-2014/094173      WO-A1-2014/103954
WO-A1-2014/178806      WO-A1-2015/153913
WO-A1-2016/205950      US-A1- 2009 137 908
US-A1- 2010 185 064      US-A1- 2011 105 865
US-A1- 2011 206 254      US-A1- 2012 277 559
US-A1- 2013 012 794      US-A1- 2013 273 524
US-A1- 2014 018 647      US-A1- 2014 043 519
US-A1- 2014 132 781

• NORIMICHI TSUMURA ET AL: "Independent-
component analysis of skin color image",
JOURNAL OF THE OPTICAL SOCIETY OF
AMERICA A, vol. 16, no. 9, 1 September 1999
(1999-09-01), pages 2169, XP055000995, ISSN:
1084-7529, DOI: 10.1364/JOSAA.16.002169

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

## Description

### Technical Field

Method and apparatus consistent with exemplary embodiments relates to an image capturing device and a sensing protection device.

### Background Art

An optical approach is an optimal solution to measuring a state of human skin according to a lot of researches on measuring chromophores and malformations of human skin. The optical solution is simple, in which a light source is used to emit light on a region of skin and reflected light is analyzed to measure a distribution of chromophores and a malformation of the skin.

This aforementioned optical approach relies on two techniques. First, the technique of precisely measuring light reflected from the skin and generating related color spectrums has been developed. Second, the technique of determining intrinsic optical parameters ma, ms, g, and n of the skin and mathematically modeling a light propagation process in a tissue has been developed. Light reflected in a backward direction contain information regarding various elements of a skin (for example, melanin, hemoglobin, water, carotene, and bilirubin). Reflectance spectroscopy is a well-known approach for distinguishing chromophores of a skin from each other and measuring a meaningful distribution of a specific chromophore in a skin according to the concentration thereof. In this approach, lights having different wavelengths are emitted on a test region of a skin. The lights are emitted on the skin and interact with the chromophores of the skin according to the qualities of the elements of the skin or scattering of the lights. The difference between the spectrums of images of a skin caused by ultraviolet radiation has been used to detect damages to the skin caused by sunlight in early stages.

Among the human senses, sight and color perception are perhaps the most interesting. With the digital revolution, color has become even more accessible. Among the optical methods of skin investigation, colorimetry is one of the most advanced.

Colorimetry has been used as an objective measure of perceived skin color by human eye to detect and score physiological responses of the skin from the various external stimulus.

Relevant prior art is disclosed in WO 2014/178806, US 2014/018647, WO 2014/094173,US 2009/137908 and US 2013/012794.

### Disclosure of Invention

### Technical Problem

Devices for measuring chromophores and malformation of a human skin are relatively huge in size, expensive due to their optics elements, and have limited data processing ability because of the restrictions of their internal processing units or necessity to use external data processing units (e.g., a PC).

### Solution to Problem

Provided are an image capturing device which is handy and is capable of measuring a state of a skin by maintaining a measurement environment constant, and a sensing protection device configured to be attachable to and detachable from the image capturing device.

### Advantageous Effects of Invention

According to the exemplary embodiments set forth herein, a state of a skin may be conveniently measured using a mobile device.

According to the exemplary embodiments set forth herein, a state of the skin may be reliably measured by maintaining a measurement environment constant.

### Brief Description of Drawings

These and/or other aspects will become apparent and more readily appreciated from the following description of the exemplary embodiments, taken in conjunction with the accompanying drawings in which:

FIG. 1 is a diagram schematically illustrating an image capturing device according to an exemplary embodiment;

FIG. 2 is a diagram schematically illustrating an image capturing device according to an exemplary embodiment;

FIG. 3 is a diagram schematically illustrating an image capturing device according to an exemplary embodiment;

FIG. 4 illustrates an algorithm of receiving light reflected from a skin and image-processing the reflected light, according to an exemplary embodiment;

FIG. 5 is a diagram illustrating a sensing protection device using a magnet as a connection unit according to an exemplary embodiment;

FIG. 6 is a diagram illustrating a sensing protection device using a slide as a connection unit according to an exemplary embodiment;

FIG. 7 is a diagram illustrating a sensing protection device using a case as a connection unit according to an exemplary embodiment; and

FIG. 8 is a diagram illustrating a sensing protection device using a leg as a connection unit according to an exemplary embodiment.

## Best Mode for Carrying out the Invention

Additional aspects will be set forth in part in the description which follows and, in part, will be apparent from the description, or may be learned by practice of the presented exemplary embodiments.

The present invention is defined by the independent claims. Further aspects of the present invention are outlined in the dependent claims. According to a first aspect of the present invention there is provided an image capturing device comprising;

a light source configured to emit light on a skin;

an image sensor configured to obtain an image of the skin from lights reflected from the skin;

a sensing protection device configured to surround the light source and the image sensor, the sensing protection device including a connection unit configured to be attached to and detached from the image capturing device; and

a controller configured to:

determine three-color coordinates of an image obtained in use of light from the light source reflected from a skin;

perform mathematical modeling to model light propagation in the skin to simulate a reflectance spectrum of skin in the visible spectrum range based on modeled concentrations of chromophores in the skin;

compare the three-color coordinates with comparing data obtained by performing the mathematical modeling;

determine concentrations of chromophores of the skin, based on a comparison of the three-colour coordinates with the comparing data obtained by performing the mathematical modeling,

wherein the mathematical modeling is performed using a Monte Carlo algorithm,

wherein the comparing data includes information about concentration of melanin and blood in a skin.

According to a further aspect of the present invention there is provided method of determining contents of chromophores of a skin by using a portable skin chromophores measuring device, the method comprising:

generating measurement conditions beforehand by using an opaque material of a sensing protection device surrounding a white-light source and an image sensor;

emitting light using the white-light source of the portable skin chromophores measuring device;

receiving light reflected from a skin by using an optical system of the portable skin chromophores measuring device;

detecting the reflected light and generating at least one image by using the image sensor of the portable skin chromophores measuring device;

determining three-color coordinates of each of the at least one image by using a processor of the portable skin chromophores measuring device;

performing mathematical modeling to simulate a radio wave of a visible spectrum range in a biological tissue by using the processor of the portable skin chromophores measuring device, based on the measurement conditions;

comparing the three-color coordinates with a result of performing the mathematical modeling; and

determining contents of the chromophores of the skin, based on a reflected spectrum of the skin.

## Mode for the Invention

Reference will now be made in detail to exemplary embodiments, examples of which are illustrated in the accompanying drawings, wherein like reference numerals refer to like elements throughout. Also, a structure of an electronic device and a method of operating the electronic device according to an exemplary embodiment will be described in detail with reference to the accompanying drawings.

It will be understood that, although the terms "first," "second," etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the scope of the inventive concept. The term 'and/or' includes any and all combinations of one or more of the associated listed items.

The terminology used herein is for the purpose of describing particular embodiments only and is not intended to limit of the inventive concept. As used herein, the singular expression does not limit the present disclosure to have a singular component or step. Instead, the present disclosure may comprise multiple components or steps even it is described in a singular express. It will be further understood that the terms 'comprise' and/or 'comprising,' when used in this specification, specify the presence of stated features, integers, steps, operations, elements, and/or components, but do not preclude the presence or addition of one or more other features, integers, steps, operations, elements, components, and/or groups thereof.

As used herein, expressions, such as "at least one of," when preceding a list of elements, modify the entire list of elements and do not modify the individual elements of the list.

Hereinafter, exemplary embodiments will be described with reference to the accompanying drawings.

In order to direct the aspects of the exemplary embodiments described above, an image capturing device including an opaque sensing protection device providing conditions required to detect light scattered in a backward direction, a white-light source for covering a visible spectrum domain from 350 nm to 800 nm and unidirectional radiation, and a color photosensitive element array configured to detect scattered light are used. Also, it is assumed that any portion of a human body (a change in a skin type, a change in a thickness of a layer of fat, etc.) can be tested and a test region is large, e.g., 10 cm2. When the sensing protection device is used, an image may be detected at any time in the same illumination state and the same detection mode. Thus, the color coordinates of an image may be associated with an actual physical wavelength in a visible region.

Because the sensing protection device may have an arbitrary shape and a telescopic function, the sensing protection device may be used in a mobile device including a flash and a camcorder. Owing to an opaque material of the sensing protection unit, the concentration of the chromophores of the skin may be measured under different kinds of external conditions (e.g., under natural light, under artificial light, when the amount of light is insufficient, etc.). A reference white color (e.g., BaSO4) of an image may be normalized to compensate for instability of a light source (a change in a battery potential, a change in a source spectrum intensity, dust, etc.). A versatile property of an exemplary embodiment applicable to various types of platforms is secured because the height of the sensing protection device may be changed (a telescopic function).

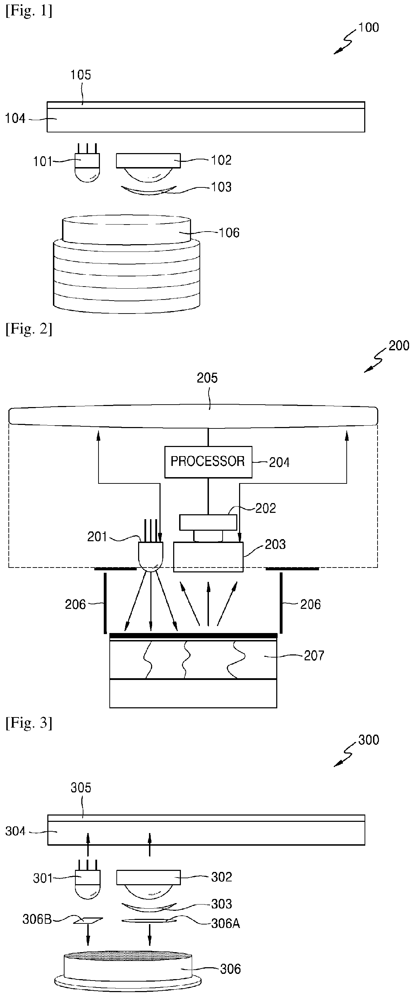

FIG. 1 is a diagram schematically illustrating an image capturing device 100 according to an exemplary embodiment.

Referring to FIG. 1, the image capturing device 100 includes a light source 101, an image sensor including a lens 103 and a photosensitive element array 102, a processor 104, a display 105, and a sensing protection device 106.

The light source unit 101 emits white light on a skin. The image sensor 102 detects scattered light. The processor 104 converts the detected light into an electrical signal. The electrical signal may be a digital image. The electrical signal is processed according to an image processing algorithm determined beforehand by the processor 104 and is then displayed on the display 105.

The sensing protection device 106 may provide detection conditions, e.g., an angle between a detector/light source and a region to be tested, exclusion of ambient light, etc.

The sensing protection device 106 may be opaque. The opaque property of the sensing protection device 106 may be an important factor that defines measurement conditions. When the sensing protection device 106 is opaque, ambient light may be excluded and this factor may be taken into account during a light transfer calculation.

The sensing protection device 106 has an arbitrary shape and a telescopic function. The inside of the sensing protection device 106 may be coated with a light-absorbing material. The type of a coated surface (e.g., a reflective type or absorbing type) of the sensing protection device 106 is an important parameter in light transfer calculation because the contents of chromophores of the skin depend on the type and current state of the skin. When the coated surface is the absorbing type, the color of a final skin image is changed by randomly reflected lights and thus all optical components reflected again may be excluded. When the coated surface is the reflective type, contribution of all components reflected may be taken into account, which may cause a calculation algorithm to be changed. The reflective or absorbing characteristic of the coated surface are characteristics of an anode that reflects or absorbs light. In the exemplary embodiments set forth herein, reflective or absorbing characteristics of a sensing protection device may be taken into account.

In the exemplary embodiments set forth herein, a reflection coefficient may be set to be in a visible range that does not exceed 4% to 5% when an inside of a sensing protection device is coated with an absorbing material, and may be set to be in a visible range that does not exceed 95% to 99% when an inside of a sensing protection device is coated with a reflective material.

A reference image of a standard white color (e.g., BaSO4) may be normalized in order to compensate for instability of a light source (a change in a battery potential, a change in a source spectrum intensity, a change in a state of a protection coating layer, dust, etc.). Small patterns, such as dots or other skin formations, are ignorable. Effects of a change in a battery potential, a change in a source spectrum intensity, dust, etc. may be also ignorable.

According to an exemplary embodiment, the sensing protection device 106 may have an arbitrary shape and the height thereof may be determined by an initial focal length of a camera lens of the image capturing device 100 and requirements for a field of view (i.e., a region of a skin to be tested). Homeostasis of melanin and red spots cannot be measured using the image capturing device 100 without the sensing protection device 106 because a process of measuring homeostasis of melanin and red spots may need to be performed under specific conditions, such as a specific angle of capturing, detection of an under-exposure state, etc.

According to an exemplary embodiment, a back focal length of an optical system employed in the image capturing device 100 may be decreased to decrease the height of the sensing protection device 106. The back focal length means the distance from a surface to a back focus. The height of the sensing protection device 106 may be decreased to 0.5 cm. To this end, a focal reducer may be added to the image capturing device 100. In this embodiment, the focal reducer is a positive lens in which a decrease in a focal distance may be calculated by the following equation:

$$R=1-D/FR,$$

wherein "R" denotes a decreased focal distance coefficient, "D" denotes a distance to an image plane, and "FR" denotes a focal distance of a lens. For example, when a lens having a focal distance of 100 mm is located at a distance of 20 mm from the image plane, the focal distance may decrease by 0.8 (=1-20/100). An achromatic lens may be additionally used to detect macro-images.

The image capturing device 100 may be a device including a light source and an image sensor to emit light and detect reflected light from the emitted lights and including a processor to process the detected light. Examples of the image capturing device 105 may include a mobile phone, a smart phone, a smart watch, a tablet device, a notebook device, etc.

As described above, the sensing protection device 106 capable of providing measurement conditions may be included in the image capturing device 100. The sensing protection device 106 may be a portable device to decrease a measurement error caused by an external optical condition. Also, device usability may be increased using a small-sized portable device, and a region to be measured may be easily located by dynamically configuring the device. Also, the contents of chromophores may be monitored and an image capturing angle may be maintained constant regardless of the type of external light, under any environment in which external light is not present, or regardless of the type of the skin.

FIG. 2 is a diagram schematically illustrating an image capturing device 200 according to an exemplary embodiment.

Referring to FIG. 2, the image capturing device 200 includes a light source 201, a photosensitive element array 202, a camera lens 203, a processor 204, a display 205, and a sensing protection device 206.

A skin 207 is prepared. Data regarding the contents of melanin and hemoglobin in the skin 207 may be obtained by performing two operations, i.e., defining the color coordinates of an image of the skin 207 and comparing an obtained value with a result obtained by performing mathematical modeling. A color value may be the same as a specific ratio between the concentrations of the melanin and the hemoglobin.

FIG. 3 is a diagram schematically illustrating an image capturing device 300 according to an exemplary embodiment.

Referring to FIG. 3, the image capturing device 300 includes a light source 301, an image sensor which is a photosensitive element array 302 including a lens 303 and a sensing protection device 306, a processor 304, and a display 305.

The image sensor further includes optical elements 306A and 306B and may thus decrease the height of the sensing protection device 306 by decreasing a focal length of a sensing unit. The sensing protection device 306 is embodied as an optical element. The lens 306A decreases a focal length of a camera of the image capturing device 300 and thus the sensing protection device 306 may be manufactured in a very small size. The scattering plate 306B enables regular surface radiation. The lens 306A and the scattering plate 306B may be integrally formed with the sensing protection device 306. The sensing protection device 306 may include a cover, and the inside of which has a reference white color.

As described above, the spectrum of a skin may be obtained from a user's digital photograph by using known color coordinates of an image of a region of the skin.

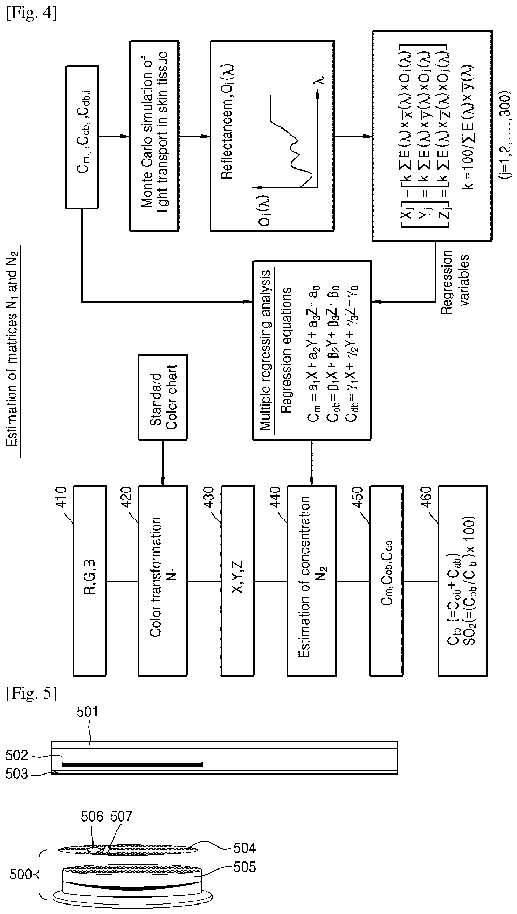

FIG.4 illustrates an algorithm of receiving lights reflected from a skin and processing the reflected light, according to an exemplary embodiment.

Various algorithms may be used to determine the color coordinates of an image of the skin, compare obtained color values with data of the skin obtained through a simulation, and estimate the concentrations of hemoglobin and melanin in the skin.

A surface of a device on which a sensing protection device is located is placed on a region of the skin to be measured. Next, a user captures several images of a surface of the skin by using special software (SSW).

Then, using special software (SSW), the user takes a few shots of the skin surface.

The white light source radiates irradiation that is scattered by skin and focused by the optical system of a mobile device. Quantitative (brightness) and quality (color) color characteristics of the skin are determined using colorimetric systems. Three color coordinates (R, G, B) 410 are determined per pixel for each skin image using a colorimetric system for measuring skin reflectance. Then received coordinates are converted into color coordinates X, Y, Z 430 of MKO1931 (Yxy) colorimetric system and L *, a * and b * of MKO1976 (L * a * b *) colorimetric system using simple ratios. The content of hemoglobin and melanin in the human skin is estimated using an algorithm based on the inverse Monte Carlo simulation. At least two-layer model of the skin is adopted. The reflection coefficient of the skin is calculated using the Monte Carlo method in the spectral visible range. Then, the spectrum is recalculated in the color coordinate system and compared with experimentally obtained data of skin reflectance and thena match of color coordinates with its corresponding spectrum may be found. In this way, the content of melanin and hemoglobin in the skin can be estimated.

In that SSW, the RGB-values are converted into the tristimulus values in color space which is a device-independent color system and compatible with the common RGB working spaces (NTSC, sRGB, etc). Monte Carlo simulation (MCS) of light transport for human skin model is used to specify a relation among the tristimulus XYZ-values and the concentrations of melanin and blood.

Spectral composition of lights penetrating through biological tissues depends on the concentration and spatial distribution of chromophores within the given tissue, as well as on particular experimental conditions, including the probe geometry and multiple parameters of the incident optical radiation.

In this embodiment, the algorithm is a computational technique specially developed for simulation of the visible reflectance spectra of human skin and skin color calculation. The computational data are compared with the experimental data obtained using high-dynamic range measurements through different parts of a human body, which are easily accessible through those measurements. Owing to a wide range of actual probing conditions and a very complex composite structure of tissues, no general analytical solution is available for simulating the detected scattered optical radiation and its interaction with tissues, their structural malformations and/or physiological changes. Therefore, stochastic Monte Carlo (MC) modeling was employed. In this embodiment, an object oriented MC model is utilized. The object oriented MC model may describe photons and tissue structural components as objects, which interact each other. Thus, an object photon propagates through an object medium (or medium layer) and interacts with its constituents, such as cells, blood vessels, collagen fibers, etc. Such representation of the medium by objects makes it possible to develop realistic tissue models presenting 3D spatial variations of a biological structure. To simulate transmission spectra, a multilayered tissue model is applied. The model known from a prior art was extended to 17 layers by including muscles and bone structures.

The MC simulations were performed taking into account at least the actual probe geometry used in the experiment utilizing the detected photon packets. The conversion of the spectral distribution to the CIE XYZ (CIE 1976 L*a*b*) coordinates, as well as to the RGB color values, was performed using the standard CIE 2° observer and tristimulus values utilizing Galaxy Note 4 WLED illuminant. Observing the effect of the changes of tissues color due to, for example, changes of blood and/or melanin content, and variations in blood oxygenation, is of a potential use for the practical diagnostic purpose and bioengineering applications. These changes can be quantified and characterized with the developed MC model.

The main provisions of the proposed method are as follows: skin imaging in constant conditions of light detecting is provided by using specially developed lens hood; the numerical experiment of light transport in skin is performed for known conditions of light detecting and light source and results are the number of spectrums and corresponding color coordinates for different content of chromophores in skin; the captured color coordinates are compared with color coordinates and corresponded spectrums obtained with the numerical modeling.

The comparing data (numerically obtained) already contain the information about concentration of melanin and blood for particular skin image. So when a conformity is found after coordinates comparison, it is possible to find the chromophores concentration.

Relations between RGB-values and skin chromophore concentrations are as follows.

RGB values of a pixel on a skin surface image acquired by a digital camera can be expressed as:

$$\begin{pmatrix} R \\ G \\ B \end{pmatrix}^T = L_1 \times \begin{pmatrix} X \\ Y \\ Z \end{pmatrix}^T$$

Where

$$X = \int\limits_{400}^{700} E(\lambda)\,\overline{x}(\lambda)O(\lambda)d(\lambda)$$

$$Y = \int\limits_{400}^{700} E(\lambda)\,\overline{y}(\lambda)O(\lambda)d(\lambda)$$

$$Z = \int\limits_{400}^{700} E(\lambda)\,\overline{z}(\lambda)O(\lambda)d(\lambda)$$

$\begin{pmatrix} X \\ Y \\ Z \end{pmatrix}$ are tristimulus values in CIEXYZ color system and $(\cdots)^T$ represents transposition of a vector. $L_1$ is a transformation matrix to convert XYZ values to the corresponding RGB values and it is available for each working space (NTSC, PAL/SECAM, sRGB, etc). $\lambda$, $E(\lambda)$, and $O(\lambda)$ are wavelength, spectral distribution of illuminant (absolute Galaxy diode spectrum, photometric), and the diffuse reflectance spectrum of human skin, respectively. $x(\lambda)$, $y(\lambda)$, and $z(\lambda)$ are color matching functions in the CIEXYZ color system. Integrals are executed over the visible wavelength range (400 to 700 nm). Assuming that the skin tissue mainly consists of epidermis containing the melanin and dermis containing blood, the diffuse reflectance of skin tissue O can be expressed as:

$$O = \frac{I}{I_0} = \left[ \int_0^\infty P_e\left(\mu_{s,e}, g_e, l_e\right) exp\left(-\mu_{a,m} l_e\right) dl_e \right] \times \left[ \int_0^\infty P_d\left(\mu_{s,d}, g_d, l_d\right) exp\left(-\mu_{a,b} l_d\right) dl_d \right]$$

where, $I_0$ and $I$ are standard reflected light and detected light intensities, respectively. $P(\mu_s, g, l)$ is the path length probability function that depends on the scattering properties as well as on the geometry of the measurements. $\mu_s$, $\mu_a$, $g$, and $l$ are the scattering coefficient, absorption coefficient, anisotropy factor, and photon path length, respectively. Subscripts, m, b, e, and d indicate melanin, blood, epidermis, and dermis, respectively. The absorption coefficient of each chromophore is expressed by the product of its concentration C and extinction coefficient $\varepsilon$ and thus $\mu_a = C_\varepsilon$. Therefore, the RGB values are expressed as the functions of Cm, and Cb.

In estimating skin chromophores concentrations based on RGB image, the following procedure is used. At first, the RGB values in each pixel of the skin image are transformed into XYZ values by a matrix $N_1$ as:

$$\begin{pmatrix} R \\ G \\ B \end{pmatrix} = N_1 \times \begin{pmatrix} X \\ Y \\ Z \end{pmatrix}$$

(in each pixel of the image)

Then, the matrix $N_1$ should be determined based on measurements of Color Checker standard that has 24 color chips and it is supplied with data giving the CIEXYZ values for each chip under the specific illumination and corresponding reflectance spectra. The values of X, Y, and Z are then transformed into Cm, Cb by a matrix N2. Then, diffuse reflectance spectra $O(\lambda)$ in a wavelength range from 400 to 700 nm at intervals of 5 nm by the MCS for light transport in skin tissue under various values of Cm, Cb is calculated. Then, the corresponding X, Y, and Z are obtained. In this MCS, the absorption coefficient of melanin for Cm is input to the epidermis as $\mu_a$, m. The absorption coefficient of blood for Cb is input to dermis as $\mu_a$ and db. The layer thickness of epidermis and dermis are set as 0.05 mm and 5.05 mm, respectively. Refractive index

EP 3 235 242 B1

for each layer is assumed to be 1.4. The XYZ-values are then calculated based on the simulated O(λ). The above calculations are executed for various combinations of Cm, Cob, and Cdb to obtain the data sets of chromophore concentrations and XYZ-values. The multiple regression analysis with data sets established the two regression equations for Cm, and Cb as:

$$Cm = a_0 + a_1 X + a_2 Y + a_3 Z,$$

$$Cb = b_0 + b_1 X + b_2 Y + b_3 Z,$$

The regression coefficients ai, bi (i=0, 1, 2, 3) reflect the contributions of XYZ-values to Cm, and Cb, respectively, and are used as the elements of a 4-by-3 matrix $N_2$. Transformation with $N_2$ from the tristimulus values to the chromophore concentrations is thus expressed as:

$$\begin{pmatrix} C_m \\ C_b \end{pmatrix} = N_2 \times \begin{pmatrix} X \\ Y \\ Z \\ 1 \end{pmatrix}$$

Once the matrices $N_1$ and $N_2$ are determined, images of Cm, and Cb are reconstructed without the MCS. The proposed technique differs from existing solutions by adding unique experiment conditions and by adding unique total spectrum of Galaxy diode.

An image capturing device may display information regarding the contents of the chromophores of a region of the skin to be tested within several seconds after lights are emitted on the skin by using a sensing protection device and an image processing algorithm as described above.

According to an exemplary embodiment, the image capturing device may further display color information of the region of the skin, a warning about a possibility that a user will suffer from sunburn, a tip for protecting the user's skin, etc.

A method of providing a user with information and the types of information to be provided to the user may be variously determined. A color density may be presented using a value or a two-dimensional (2D) color distribution map.

According to an exemplary embodiment, information regarding a state of the skin may be transmitted to a doctor or a cosmetician. For example, information regarding a medical institution may be registered with an image capturing device, and the image capturing device may automatically transmit information regarding a state of the skin to the registered medical institute whenever the image capturing device measures the state of the skin and obtains measurement data.

Various methods may be used to attach a sensing protection device to the image capturing device.

The image capturing device and the sensing protection device include various connection units.

According to an exemplary embodiment, a connection unit connecting the image capturing device and the sensing protection device may include a magnet.

FIG. 5 is a diagram illustrating a sensing protection device 500 using a magnet as a connection unit according to an exemplary embodiment.

Referring to FIG. 5, a metal plate 502 is located behind a rear-surface cover 503 of an image capturing device 501. In this case, the sensing protection device 500 may include the metal plate 502 and a magnet unit 504 as parts of the sensing protection device 500. The sensing protection device 500 may be attached to the image capturing device 501 via the magnet unit 504.

The magnet unit 504 may include a hole 506 through which a light source passes, and a hole 507 through which light reflected from the skin passes.

According to an exemplary embodiment, a connection unit connecting an image capturing device and the sensing protection device 500 may include a slide.

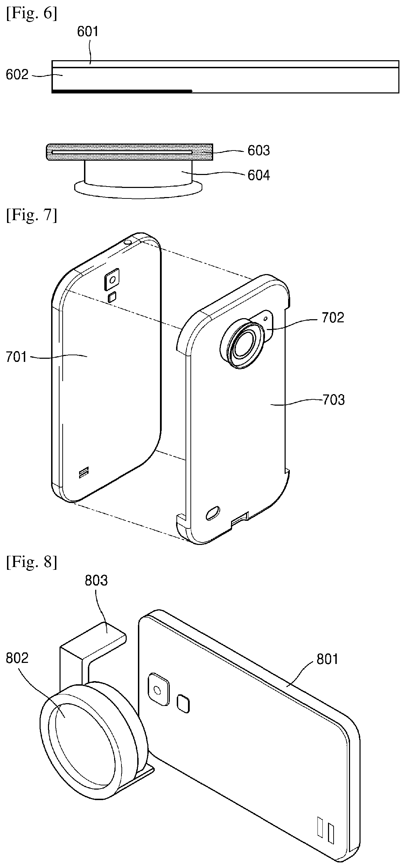

FIG. 6 is a diagram illustrating a sensing protection device 604 using a slide as a connection unit according to an exemplary embodiment.

Referring to FIG. 6, the sensing protection device 604 includes, as connection units, a slide groove 602 and a slide 603 to fix the sensing protection device 604 onto an image capturing device 601.

The sensing protection device 604 and the image capturing device 601 may be connected by inserting the slide 603 included in the sensing protection device 604 into the side groove 602 formed in a rear surface of the image capturing device 601 such that the slide 603 is moved into the slide groove 602 in a sliding manner.

According to an exemplary embodiment, a connection unit connecting the image capturing device 601 and the sensing protection device 604 may include a case.

FIG. 7 is a diagram illustrating a sensing protection device 702 using a case as a connection unit according to an exemplary embodiment.

Referring to FIG. 7, the sensing protection device 702 may be included in a phone case 703. In this case, the sensing protection device 702 may be a part of the plastic case 703. The image capturing device may be fixed on the case 701. Therefore, the image capturing device and the sensing protection device may be connected by fixing the case 701 with the case 703.

According to an exemplary embodiment, a connection unit connecting an image capturing device and a sensing protection device may include a leg.

FIG. 8 is a diagram illustrating a sensing protection device 802 using a leg as a connection unit according to an exemplary embodiment.

Referring to FIG. 8, the sensing protection device 802 may include a leg 803 for attaching the sensing protection device 802 to an image capturing device 801.

The image capturing devices according to the exemplary embodiments described above may be used as devices for predicting the contents of melanin and blood of a user's skin. The user may estimate a tanning time and predict a tanning level in the near future, based on information regarding the contents of the melanin and blood. Also, the image capturing devices may be used to estimate absolute color values of any opaque solid-type material.

The above exemplary embodiments are applicable to measuring the optical characteristics of a skin to perform a self-tanning treatment by appropriately radiating light on the skin.

The above exemplary embodiments are related to the field of medicine or skin science. A diagnosis of a state of a skin may be used to obtain objective information regarding a spatial distribution and contents of melanin, hemoglobin, and other bio-molecules in a tissue.

A quantitative estimation of optical parameters of a human skin may provide important information to a dermatologist, and may be used in the field of skin disease diagnostics or chemistry or used to investigate influences caused by environmental factors such as UV radiation, temperature, etc.

According to the exemplary embodiments set forth herein, a state of a skin may be conveniently measured using a mobile device.

According to the exemplary embodiments set forth herein, a state of the skin may be reliably measured by maintaining a measurement environment constant.

The methods of operating an electronic device described herein may be written as program commands executable via any computer means and recorded in a computer-readable recording medium. The computer-readable recording medium may include a program command, a data file, and a data structure solely or in combination. Program commands recorded in the computer-readable recording medium may be specifically designed and configured for the inventive concept, or may be well known to and usable by one of ordinary skill in the art of computer software. Examples of the computer-readable recording medium include magnetic media (e.g., hard disks, floppy disks, and magnetic tapes), optical media (e.g., CD-ROMs and DVDs), magnetooptical media (e.g., floptical disks), and hardware devices specifically configured to store and execute program commands (e.g., ROMs, RAMs, and flash memories). Examples of program commands include not only machine language codes prepared by a compiler, but also high-level language codes executable by a computer by using an interpreter.

It should be understood that exemplary embodiments described herein should be considered in a descriptive sense only and not for purposes of limitation. Descriptions of features or aspects within each exemplary embodiment should typically be considered as available for other similar features or aspects in other exemplary embodiments.

## Claims

1. An image capturing device comprising;

   a light source (101, 201, 301) configured to emit light on a skin;
   an image sensor configured to obtain an image of the skin from lights reflected from the skin;
   a sensing protection device (106, 206, 306) configured to surround the light source and the image sensor, the sensing protection device including a connection unit configured to be attached to and detached from the image capturing device; and
   a controller (104, 204, 304) configured to:

determine three-color coordinates of an image obtained in use of light from the light source reflected from a skin;

perform mathematical modeling to model light propagation in the skin to simulate a reflectance spectrum of skin in the visible spectrum range based on modeled concentrations of chromophores in the skin;

compare the three-color coordinates with comparing data obtained by performing the mathematical modeling;

determine concentrations of chromophores of the skin, based on a comparison of the three-colour coordinates with the comparing data obtained by performing the mathematical modeling,

wherein the mathematical modeling is performed using a Monte Carlo algorithm,

wherein the comparing data includes information about concentration of melanin and blood in a skin.

2. The image capturing device of claim 1, wherein an inside of the sensing protection device comprises a light-absorbing material.

3. The image capturing device of claim 1, further comprising a light sensing element and a white-light source.

4. The image capturing device of claim 1, wherein the sensing protection device is further configured to provide a telescopic function.

5. The image capturing device of claim 1, wherein a height of the sensing protection device is extensible according to a focal length of a camera.

6. The image capturing device of claim 1, wherein the sensing protection device has a height determined by a focal length of the image capturing device.

7. The image capturing device of claim 1, wherein the connection unit of the sensing protection unit comprises at least one among a magnet, a slide, and a case.

8. The image capturing device of claim 1, wherein the sensing protection device comprises:

a sensing protection member configured to surround a light source and an image sensor included in an image capturing device; and

a connection member configured to connect the sensing protection member to the image capturing device such that the sensing protection member is attachable to and detachable from the image capturing device,

wherein light is emitted on a skin from the light source of the image capturing device via the sensing protection member, and an image of the skin generated from light reflected from the skin is captured by the image sensor.

9. The image capturing device of claim 1, wherein the connection unit comprises:

a first connecting unit being located on the image capturing device, and

a second connecting unit being located on the sensing protection device, the first connecting unit and the second connecting unit being configured to be connected/disconnected with each other such that the sensing protection device is capable of being attached to/detached from the image capturing device.

10. The image capturing device of claim 8, wherein the first connecting unit is a metal plate and the second connecting unit is a magnet.

11. The image capturing device of claim 8, wherein the first connecting unit is a slide groove and the second connecting unit is a slide.

12. The image capturing device of claim 8, wherein the first connecting unit is a first case, the second connecting unit is a second case, and the first case and the second case are capable of being fixed with each other.

13. A method of operating an image capturing device for determining contents of chromophores of a skin, the method comprising:

generating measurement conditions beforehand by using an opaque material of a sensing protection device surrounding a white-light source and an image sensor;

emitting light using the white-light source of the image capturing device;

receiving light reflected from a skin by using an optical system of the image capturing device;

detecting the reflected light and generating at least one image by using the image sensor of the image capturing device;

determining three-color coordinates of each of the at least one image by using a controller of the image capturing device;

performing mathematical modeling to model light propagation in the skin to simulate a reflectance spectrum of skin in the visible spectrum range based on modeled concentrations of chromophores in the skin by using the controller of the image capturing device, based on the measurement conditions;

comparing the three-color coordinates with comparing data obtained by performing the mathematical modeling; and

determining concentrations of the chromophores of the skin, based on the comparison of the three-colour coordinates with the comparing data obtained by performing the mathematical modeling,

wherein the mathematical modeling is performed using a Monte Carlo algorithm,

wherein the comparing data includes information about concentration of melanin and blood in a skin.

14. The method of claim 13, wherein a skin model used in the Monte Carlo algorithm varies according to a portion of a body.

15. The method of claim 13, wherein at least one of a structure of the skin, parameters of an experiment, measurement elements, sizes and shapes of incident light and detected light is taken into account in the Monte Carlo algorithm.

**Patentansprüche**

1. Bilderfassungsvorrichtung, umfassend;

eine Lichtquelle (101, 201, 301), die dazu konfiguriert ist, Licht auf einer Haut zu emittieren;

einen Bildsensor, der dazu konfiguriert ist, ein Bild der Haut aus Licht zu erhalten, das von der Haut reflektiert wird;

eine abtastende Schutzvorrichtung (106, 206, 306), die dazu konfiguriert ist, die Lichtquelle und den Bildsensor zu umgeben, wobei die abtastende Schutzvorrichtung eine Verbindungseinheit beinhaltet, die dazu konfiguriert ist, an der Bilderfassungsvorrichtung angebracht und davon gelöst zu sein; und

eine Steuerung (104, 204, 304), die zu Folgendem konfiguriert ist:

Bestimmen von Dreifarbenkoordinaten eines Bildes, das in Verwendung von Licht von der Lichtquelle, das von einer Haut reflektiert wird, erhalten wird;

Durchführen von mathematischer Modellierung, um Lichtausbreitung in der Haut zu modellieren, um ein Reflexionsspektrum von Haut in dem sichtbaren Spektralbereich basierend auf modellierten Konzentrationen von Chromophoren in der Haut zu simulieren;

Vergleichen der Dreifarbenkoordinaten mit Vergleichsdaten, die durch Durchführen der mathematischen Modellierung erhalten werden;

Bestimmen von Konzentrationen von Chromophoren der Haut basierend auf einem Vergleich der Dreifarbenkoordinaten mit den Vergleichsdaten, die durch Durchführen der mathematischen Modellierung erhalten werden,

wobei die mathematische Modellierung unter Verwendung eines Monte-Carlo-Algorithmus durchgeführt wird,

wobei die Vergleichsdaten Informationen über Konzentration von Melanin und Blut in einer Haut beinhalten.

2. Bilderfassungsvorrichtung nach Anspruch 1, wobei eine Innenseite der abtastenden Schutzvorrichtung ein lichtabsorbierendes Material umfasst.

3. Bilderfassungsvorrichtung nach Anspruch 1, ferner umfassend ein lichtabtastendes Element und eine Weißlichtquelle.

4. Bilderfassungsvorrichtung nach Anspruch 1, wobei die abtastende Schutzvorrichtung ferner dazu konfiguriert ist, eine Teleskopfunktion bereitzustellen.

5. Bilderfassungsvorrichtung nach Anspruch 1, wobei eine Höhe der abtastenden Schutzvorrichtung gemäß einer Brennweite einer Kamera erweiterbar ist.

6. Bilderfassungsvorrichtung nach Anspruch 1, wobei die abtastende Schutzvorrichtung eine Höhe aufweist, die durch eine Brennweite der Bilderfassungsvorrichtung bestimmt ist.

7. Bilderfassungsvorrichtung nach Anspruch 1, wobei die Verbindungseinheit der abtastenden Schutzeinheit zumindest eines von einem Magneten, einem Schlitten und einem Gehäuse umfasst.

8. Bilderfassungsvorrichtung nach Anspruch 1, wobei die abtastende Schutzvorrichtung Folgendes umfasst:

ein abtastendes Schutzelement, das dazu konfiguriert ist, eine Lichtquelle und einen Bildsensor zu umgeben, die in einer Bilderfassungsvorrichtung beinhaltet sind; und
ein Verbindungselement, das dazu konfiguriert ist, das abtastende Schutzelement mit der Bilderfassungsvorrichtung zu verbinden, sodass das abtastende Schutzelement an der Bilderfassungsvorrichtung anbringbar und davon abnehmbar ist,
wobei Licht auf einer Haut von der Lichtquelle der Bilderfassungsvorrichtung über das abtastende Schutzelement emittiert wird und ein Bild der Haut, das aus Licht erzeugt wird, das von der Haut reflektiert wird, durch den Bildsensor erfasst wird.

9. Bilderfassungsvorrichtung nach Anspruch 1, wobei die Verbindungseinheit Folgendes umfasst:

eine erste Verbindungseinheit, die sich an der Bilderfassungsvorrichtung befindet, und
eine zweite Verbindungseinheit, die sich an der abtastenden Schutzvorrichtung befindet, wobei die erste Verbindungseinheit und die zweite Verbindungseinheit dazu konfiguriert sind, miteinander verbunden/getrennt zu sein, sodass die abtastende Schutzvorrichtung in der Lage ist, an der Bilderfassungsvorrichtung angebracht/davon gelöst zu sein.

10. Bilderfassungsvorrichtung nach Anspruch 8, wobei die erste Verbindungseinheit eine Metallplatte ist und die zweite Verbindungseinheit ein Magnet ist.

11. Bilderfassungsvorrichtung nach Anspruch 8, wobei die erste Verbindungseinheit eine Schlittennut ist und die zweite Verbindungseinheit ein Schlitten ist.

12. Bilderfassungsvorrichtung nach Anspruch 8, wobei die erste Verbindungseinheit ein erstes Gehäuse ist, die zweite Verbindungseinheit ein zweites Gehäuse ist und das erste Gehäuse und das zweite Gehäuse in der Lage sind, miteinander fixiert zu sein.

13. Verfahren zum Betreiben einer Bilderfassungsvorrichtung zum Bestimmen von Gehalten an Chromophoren einer Haut, wobei das Verfahren Folgendes umfasst:

Erzeugen von Messbedingungen im Voraus durch Verwenden eines opaken Materials einer abtastende Schutzvorrichtung, die eine Weißlichtquelle und einen Bildsensor umgibt;
Emittieren von Licht unter Verwendung der Weißlichtquelle der Bilderfassungsvorrichtung;
Empfangen von Licht, das von einer Haut reflektiert wird, durch Verwenden eines optischen Systems der Bilderfassungsvorrichtung;
Detektieren des reflektierten Lichts und Erzeugen von zumindest einem Bild durch Verwenden des Bildsensors der Bilderfassungsvorrichtung;
Bestimmen von Dreifarbenkoordinaten von jedem von dem zumindest einen Bild durch Verwenden einer Steuerung der Bilderfassungsvorrichtung;
Durchführen von mathematischer Modellierung, um Lichtausbreitung in der Haut zu modellieren, um ein Reflexionsspektrum von Haut in dem sichtbaren Spektralbereich basierend auf modellierten Konzentrationen von Chromophoren in der Haut durch Verwenden der Steuerung der Bilderfassungsvorrichtung basierend auf den Messbedingungen zu simulieren;
Vergleichen der Dreifarbenkoordinaten mit Vergleichsdaten, die durch Durchführen der mathematischen Modellierung erhalten werden; und
Bestimmen von Konzentrationen der Chromophoren der Haut basierend auf dem Vergleich der Dreifarbenkoordinaten mit den Vergleichsdaten, die durch Durchführen der mathematischen Modellierung erhalten werden, wobei die mathematische Modellierung unter Verwendung eines Monte-Carlo-Algorithmus durchgeführt wird,

wobei die Vergleichsdaten Informationen über Konzentration von Melanin und Blut in einer Haut beinhalten.

**14.** Verfahren nach Anspruch 13, wobei ein Hautmodell, das in dem Monte-Carlo-Algorithmus verwendet wird, gemäß einem Abschnitt eines Körpers variiert.

**15.** Verfahren nach Anspruch 13, wobei zumindest eines von einer Struktur der Haut, Parametern eines Experiments, Messelementen, Größen und Formen von einfallendem Licht und detektiertem Licht in dem Monte-Carlo-Algorithmus berücksichtigt wird.

**Revendications**

**1.** Dispositif de capture d'images comprenant ;

une source de lumière (101, 201, 301) conçue pour émettre de la lumière sur une peau ;
un détecteur d'images conçu pour obtenir une image de la peau à partir de lumières réfléchies par la peau ;
un dispositif de protection de détection (106, 206, 306) conçu pour entourer la source de lumière et le détecteur d'images, le dispositif de protection de détection comprenant une unité de liaison conçue pour être fixée au dispositif de capture d'images et séparée de celui-ci ; et
un dispositif de commande (104, 204, 304) conçu pour :

déterminer des coordonnées tricolores d'une image obtenue lors de l'utilisation de lumière à partir de la source de lumière réfléchie par une peau ;
réaliser une modélisation mathématique pour modéliser une propagation de lumière dans la peau afin de simuler un spectre de réflectance de la peau dans la plage du spectre visible sur la base de concentrations modélisées de chromophores dans la peau ;
comparer les coordonnées tricolores avec des données de comparaison obtenues en réalisant la modélisation mathématique ;
déterminer des concentrations de chromophores de la peau, sur la base d'une comparaison des coordonnées tricolores avec les données de comparaison obtenues en réalisant la modélisation mathématique, dans lequel la modélisation mathématique est réalisée à l'aide d'un algorithme de Monte Carlo, dans lequel les données de comparaison comprennent des informations sur une concentration de mélanine et de sang dans une peau.

**2.** Dispositif de capture d'images de la revendication 1, dans lequel un intérieur du dispositif de protection de détection comprend un matériau absorbant la lumière.

**3.** Dispositif de capture d'images de la revendication 1, comprenant en outre un élément de détection de lumière et une source de lumière blanche.

**4.** Dispositif de capture d'images de la revendication 1, dans lequel le dispositif de protection de détection est en outre conçu pour assurer une fonction télescopique.

**5.** Dispositif de capture d'images de la revendication 1, dans lequel une hauteur du dispositif de protection de détection est extensible selon une longueur focale d'une caméra.

**6.** Dispositif de capture d'images de la revendication 1, dans lequel le dispositif de protection de détection présente une hauteur déterminée par une longueur focale du dispositif de capture d'images.

**7.** Dispositif de capture d'images de la revendication 1, dans lequel l'unité de liaison de l'unité de protection de détection comprend au moins un élément parmi un aimant, une glissière et un boîtier.

**8.** Dispositif de capture d'images de la revendication 1, dans lequel le dispositif de protection de détection comprend :

un élément de protection de détection conçu pour entourer une source de lumière et un détecteur d'images compris dans un dispositif de capture d'images ; et
un élément de liaison conçu pour connecter l'élément de protection de détection au dispositif de capture d'images de sorte que l'élément de protection de détection puisse être fixé au dispositif de capture d'images et séparé de celui-ci,

dans lequel de la lumière est émise sur une peau à partir de la source de lumière du dispositif de capture d'images par l'intermédiaire de l'élément de protection de détection, et une image de la peau générée à partir de lumière réfléchie par la peau est capturée par le détecteur d'images.

9. Dispositif de capture d'images de la revendication 1, dans lequel l'unité de liaison comprend :

une première unité de liaison étant située sur le dispositif de capture d'images, et
une seconde unité de liaison étant située sur le dispositif de protection de détection, la première unité de liaison et la seconde unité de liaison étant conçues pour être reliées l'une à l'autre ou détachées l'une de l'autre, de sorte que le dispositif de protection de détection puisse être fixé au dispositif de capture d'images ou séparé de celui-ci.

10. Dispositif de capture d'images de la revendication 8, dans lequel la première unité de liaison est une plaque métallique et la seconde unité de liaison est un aimant.

11. Dispositif de capture d'images de la revendication 8, dans lequel la première unité de liaison est une rainure de glissière et la seconde unité de liaison est une glissière.

12. Dispositif de capture d'images de la revendication 8, dans lequel la première unité de liaison est un premier boîtier, la seconde unité de liaison est un second boîtier, et le premier boîtier et le second boîtier sont capables d'être fixés l'un à l'autre.

13. Procédé de fonctionnement d'un dispositif de capture d'images pour déterminer des teneurs en chromophores d'une peau, le procédé comprenant :

la génération de conditions de mesure au préalable à l'aide d'un matériau opaque d'un dispositif de protection de détection entourant une source de lumière blanche et un détecteur d'images ;
l'émission de lumière à l'aide de la source de lumière blanche du dispositif de capture d'images ;
la réception de lumière réfléchie par une peau à l'aide d'un système optique du dispositif de capture d'images ;
la détection de la lumière réfléchie et la génération d'au moins une image à l'aide du détecteur d'images du dispositif de capture d'images ;
la détermination de coordonnées tricolores de chacune de l'au moins une image à l'aide d'un dispositif de commande du dispositif de capture d'images ;
la réalisation d'une modélisation mathématique pour modéliser une propagation de lumière dans la peau afin de simuler un spectre de réflectance de la peau dans la plage du spectre visible sur la base de concentrations modélisées de chromophores dans la peau à l'aide du dispositif de commande du dispositif de capture d'images, sur la base des conditions de mesure ;
la comparaison des coordonnées tricolores avec des données de comparaison obtenues en réalisant la modélisation mathématique ; et
la détermination de concentrations des chromophores de la peau, sur la base de la comparaison des coordonnées tricolores avec les données de comparaison obtenues en réalisant la modélisation mathématique,
dans lequel la modélisation mathématique est réalisée à l'aide d'un algorithme de Monte Carlo,
dans lequel les données de comparaison comprennent des informations sur une concentration de mélanine et de sang dans une peau.

14. Procédé de la revendication 13, dans lequel un modèle de peau utilisé dans l'algorithme de Monte Carlo varie selon une partie d'un corps.

15. Procédé de la revendication 13, dans lequel au moins un élément parmi une structure de la peau, des paramètres d'une expérience, des éléments de mesure, des tailles et des formes de lumière incidente et de lumière détectée est pris en compte dans l'algorithme de Monte Carlo.

[Fig. 1]

[Fig. 2]

[Fig. 3]

[Fig. 4]

Estimation of matrices $N_1$ and $N_2$

$C_{m,j}, C_{ob,j}, C_{db,j}$

Monte Carlo simulation of light transport in skin tissue

Reflectancem, $O_j(\lambda)$

$O_j(\lambda)$

$\lambda$

$\begin{bmatrix} X_j \\ Y_j \\ Z_j \end{bmatrix} = \begin{bmatrix} k \sum E(\lambda) \times \overline{x}(\lambda) \times O_j(\lambda) \\ k \sum E(\lambda) \times \overline{y}(\lambda) \times O_j(\lambda) \\ k \sum E(\lambda) \times \overline{z}(\lambda) \times O_j(\lambda) \end{bmatrix}$

$k = 100 / \sum E(\lambda) \times \overline{y}(\lambda)$

$(j = 1, 2, \ldots, 300)$

Regression variables

Multiple regressing analysis
Regression equations

$C_m = a_1 X + a_2 Y + a_3 Z + a_0$
$C_{ob} = \beta_1 X + \beta_2 Y + \beta_3 Z + \beta_0$
$C_{db} = \gamma_1 X + \gamma_2 Y + \gamma_3 Z + \gamma_0$

Standard Color chart

410 — R, G, B

420 — Color transformation $N_1$

430 — X, Y, Z

440 — Estimation of concentration $N_2$

450 — $C_m, C_{ob}, C_{db}$

460 — $C_{tb}$ ($= C_{ob} + C_{ab}$)
$SO_2$ ($= (C_{ob} / C_{tb}) \times 100$)

[Fig. 5]

501
502
503

506 507
504
505
500

[Fig. 6]

601

602

603

604

[Fig. 7]

701

702

703

[Fig. 8]

803

802

801

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014178806 A
- US 2014018647 A
- WO 2014094173 A
- US 2009137908 A
- US 2013012794 A